# EUROPÄISCHE PATENTSCHRIFT

(11) **EP 1 180 361 B1**
(45) Veröffentlichungstag und Bekanntmachung des Hinweises auf die Patenterteilung: **22.08.2007**
(21) Anmeldenummer: 01114158.7
(22) Anmeldetag: 11.06.2001
(51) Int. Cl.: A61Q 19/10, A61Q 19/00, A61K 8/02, A61K 8/04, A61K 8/81

(54) **Hautreinigungsmittel**
Skin cleansing composition
Composition de nettoyage pour la peau

(30) Priorität: 18.08.2000 DE 10040873
(43) Veröffentlichungstag der Anmeldung: 20.02.2002
(73) Patentinhaber: Stockhausen GmbH, 47805 Krefeld (DE)
(72) Erfinder: Veeger, Marcel, 47574 Goch (DE); Klotz, Andreas, 41516 Grevenbroich (DE); Nauels, Bernd, 47906 Kempen (DE)
(74) Vertreter: Wolff, Felix

(56) Entgegenhaltungen:
- WO-A-00/17300
- CH-A- 128 236
- US-A- 2 996 432
- US-A- 5 372 804
- DATABASE WPI Section Ch, Week 198225 Derwent Publications Ltd., London, GB; Class A96, AN 1982-51272E XP002259167 NN: "Film-type packing agent" & JP 57 077611 A (KANEBO LTD), 15. Mai 1982 (1982-05-15)
- DATABASE WPI Section Ch, Week 198126 Derwent Publications Ltd., London, GB; Class A96, AN 1981-46913D XP002259168 NN: "Skin removing sheet for sunburned skin" & JP 56 053607 A (NITTO ELECTRIC IND CO), 13. Mai 1981 (1981-05-13)
- DATABASE WPI Section Ch, Week 198420 Derwent Publications Ltd., London, GB; Class A96, AN 1984-124826 XP002259169 NN: "Storage stable pack cosmetic material" & JP 59 062512 A (SHISEIDO CO. LTD.), 10. April 1984 (1984-04-10)
- NN: "Pflaster - die unauffälligen, aber unentbehrlichen Helfer" PFLEGEDIENST, [Online] Bd. 3, 2003, Seiten 10-11, XP002259165 Gefunden im Internet: <URL:http://www.hartmann-online.de/deutsch /produkte/pflege/pflegedienst/pd3_03_produ kt.pdf> [gefunden am 2003-10-24]
- DATABASE WPI Section Ch, Week 198347 Derwent Publications Ltd., London, GB; Class A18, AN 1983-823352 XP002259170 NN: "Pressure sensitive adhesive tapes or sheets" & JP 58 174484 A (KAO CORP.), 13. Oktober 1983 (1983-10-13)
- DATABASE WPI Section Ch, Week 198228 Derwent Publications Ltd., London, GB; Class A81, AN 1982-58565E XP002259171 NN: "Adhesives for artificial hair, moustache, etc." & JP 57 092073 A (SHISEIDO CO LTD), 8. Juni 1982 (1982-06-08)
- VOIGT R.: "Pharmazeutische Technologie für Studium und Beruf" 2000 , DT.APOTHEKER VERLAG , STUTTGART/BRD XP002259166 * Seite 546-547 *

## Beschreibung

Die vorliegende Erfindung betrifft ein Hautreinigunsgsmittel enthaltend ein Polyisoprenlatex.

Seit vielen Jahren sind Hautapplikationsmittel, wie z. B. Gesichtsmasken, Pflaster und Waschpasten bekannt, in denen oftmals Polymerlatices enthalten sind. Diese Hautapplikationsmittel haben jedoch oftmals den Nachteil, daß sie zu Hautreizungen führen und toxikologisch nicht unbedenklich sind.

In der WO 00/17300 wird ein Hautreinigungsmittel gelehrt, das aus einer Latexemulsion, einer Organsiliconverbindung, Emollients, oberflächenaktiven Substanzen und Wasser besteht. Die Latexemulsion weist bevorzugt ein StyrolButadien Copolymer auf.

Die oben genannten Hautreinigungsmittel haben den Nachteil, daß sie nur bedingt hautverträglich, toxikologisch nicht einwandfrei und/oder umweltgefährdend sind.

Aus der EP 0 535 789 A2 ist eine reinigende Komposition für tropische Applikationen bekannt, die eine Mischung einer oberflächenaktiven Substanz eines Emollients, eines kationischen Materials, eines Filmformers, eines Polyaminozuckerkondensats und Wasser enthält.

Es stellt sich deshalb die Aufgabe, ein Hautreinigunsgsmittel zur Verfügung zu stellen, das toxikologisch unbedenklich und das im Vergleich zu Hautreinigunsgsmittel gemäß dem Stand der Technik zu ähnlichen oder geringeren Hautreizungen führt.

Die Aufgabe wird erfindungsgemäß durch ein Hautreinigunsgsmittel gelöst, das ein Polyisoprenlatex und ein Abrasivum enthält.

Erfindungsgemäß weist das Hautreinigunsgsmittel ein Polyisoprenlatex auf. Vorzugsweise hat dieser Polyisoprenlatex einen Gehalt an cis 1, 4 Isopren von mindestens 90 Gew.-%. Ebenfalls bevorzugt beträgt die Partikelgröße der Polyisoprenteilchen im Latex maximal 1,8 µm. Weiterhin bevorzugt weist der Latex einen Feststoffgehalt von 20 bis 90 Gew.-%, vorzugsweise 60 bis 80 Gew.-%, auf.

In einer bevorzugten Ausführungsform ist das Hautreinigunsgsmittel ein kosmetisches und/oder medizinisches Hautreinigunsgsmittel jeglicher Art. Insbesondere das medizinische Hautreinigunsgsmittel weist vorzugsweise mindestens einen transdermalen Wirkstoff auf.

Vorzugsweise ist das Hautreinigungsmittel eine Waschpaste, insbesondere eine Handwaschpaste. Diese Hautreinigungsmittel können neben dem Polyisoprenlatex noch organische Lösemittel enthalten.

Vorzugsweise weist das Lösemittel eine geringere Flüchtigkeit als Ethanol auf und hat einen Siedepunkt > 78,32 °C, wobei der Siedepunkt bei einem Druck von 1,013 bar gemessen wird. Vorzugsweise ist das Lösemittel mindestens ein
- mehrwertiger Alkohol oder ein Derivat eines mehrwertigen Alkohols, insbesondere ein Polydiol, vorzugsweise ein Alkylenglykol, ein Polyalkylenglykol, ein Polydiolderivat, vorzugsweise ein Polyalkylenglykolether und/oder ein Polyalkylenglykolester,
- Mono- oder Polyester einer gesättigten oder ungesättigten ein- oder mehrwertigen Carbonsäure mit 2 bis 30 Kohlenstoffatomen mit n- und iso-Alkanolen mit 2 bis 10 Kohlenstoffatomen, vorzugsweise ein Diester von aliphatischen und/oder aromatischen Di- und/oder Tricarbonsäuren und/oder
- aliphatischer Kohlenwasserstoff mit C₁₂- bis C₂₂- Kohlenstoffatomen, vorzugsweise mit C₁₆- bis C₂₀- Kohlenstoffatomen und besonders bevorzugt Isohexadecan,
oder eine Mischung aus mindestens zwei der oben genannten Substanzen.

Bevorzugte Polyalkylenglykole oder deren Ester oder Ether sind in den Tabellen aus Ullmann, 4. Auflage, Band 8, Seiten 200, 204, 205, 207 und Band 19, Seiten 426 und 428 aufgelistet. Diese Tabellen werden hiermit als Referenz eingeführt und gelten somit als Teil der Offenbarung.

Besonders bevorzugt ist das Lösemittel Dipropylenglykolmonomethylether.

Vorzugsweise beträgt das Verhältnis von Polyisoprenlatex zu Lösemittel 8:1 bis 1:1, besonders bevorzugt 4:1 bis 2:1.

Gegebenenfalls weist das Hautreinigungsmittel ein Tensid auf. Vorzugsweise ist dieses Tensid ein Fettalkoholethoxylat der allgemeinen Formel: R-O-(CH₂-CH₂-O)ₙ mit R=C₈₋₁₈ und n=1-8, vorzugsweise R=C₁₂₋₁₄ und n=4-6 ist. Ebenfalls bevorzugt ist das Tensid ein Fettalkoholethersulfat der allgemeinen Formel: R-O-(CH₂-CH₂-O)ₙ-SO₄X₂ mit R=C₁₀₋₁₆ und n=1-4 und X= Na⁺ K⁺,½ Mg²⁺ oder NH₄⁺.

Das Hautreinigungsmittel enthält mindestens ein Abrasivum. Die Abrasiva können übliche Abrasiva oder deren Mischung, vorzugsweise gebleichtes Walnußschalenmehl, nichtquellbare Stärke oder deren Mischung sein.

Zur Beeinflussung der Konsistenz des Hautreinigungsmittels kann dieses noch wasserquellbare Polymere als Verdickungsmittel enthalten, wobei Polymere, erhältlich durch die Polymerisation von Acrylsäure sowie Xanthangum bevorzugt eingesetzt werden.

Das Hautreinigungsmittel kann kosmetische Hilfs- oder Zusatzstoffe und/oder Wirkstoffe enthalten, wie z. B. Pflegederivate, Emollients, Parfum (Duftstoffe), Konservierungsmittel u. a.

In einer bevorzugten Ausführungsform enthält das Hautreinigungsmittel:
a. 10-80 Gew.-% einer flüssigen natürlichen und/oder synthetischen wasserbasierenden Polyisoprenlatexemulsion,
b. 1-15 Gew.-% eines organischen Lösemittels, das eine geringere Flüchtigkeit als Ethanol und einen Siedepunkt > 78,32 °C aufweist,
c. 0-10 Gew.-% mindestens eines Tensides, vorzugsweise mindestens eines Fettalkoholethoxylates, Fettalkoholethersulfates, Succinates, Sarkosides und/oder Glucosides,
d. 0-10 Gew.-% Abrasiva, vorzugsweise gebleichtes Walnußschalenmehl und/oder nicht quellbare Stärke,
e. 0-1 Gew.-% Verdickungsmittel,
f. ggf. kosmetische Hilfs- und Zusatzstoffe und/oder Wirkstoffe,
g. 10-60 Gew.-% Wasser,
wobei die Summe 100 Gew.% ergibt.

Die Zusatzstoffe sind vorzugsweise reinigende und/oder pflegende Komponenten. Beispiele für reinigende Komponenten sind ionische- oder nichtionische Tenside, vorzugsweise Weizenkeimhydrolysate, Betaine, Sarkoside und/oder Sulfosuccinate.

Als pflegende Komponente werden vorzugsweise mindestens ein Vitamin, mindestens ein Kraut oder mindestens ein Bestandteil eines Krauts, mindestens eine Kräutermischung, z. B. Bisaboöl, Azulen, mindestens ein Fruchtpulver und/oder mindestens ein Bestandteil einer Frucht, insbesondere ein Vitamin, ein Fruchtpulver, und z. B. Bisabolol, Azulen und/oder Panthenol-2 und/oder gerbende Komponenten, z. B. Hamamelis, eingesetzt.

In einer anderen bevorzugten Ausführungsform der vorliegenden Erfindung ist das Hautreinigunsgsmittel ein Pflaster, insbesondere ein Sprühpflaster.

Das erfingdungsgemäße Hautreinigunsgsmittel hat den Vorteil, daß es toxikologisch unbedenklich und für die Umwelt weniger belastend ist und es die Haut zumindest nicht stärker reizt als Produkte des Standes der Technik.

Ein weiterer Gegenstand der vorliegenden Erfindung ist ein Verfahren zur medizinischen und/oder kosmetischen Behandlung der Haut, dadurch gekennzeichnet, daß ein erfindungsgemäßes Hautreinigunsgsmittel auf die Haut aufgetragen, dort über einen gewissen Zeitraum belassen und nachfolgend oder aber sofort nach dem Auftragen wieder entfernt, vorzugsweise abgerieben und/oder abgewaschen wird.

Der Zeitraum, in dem das erfindungsgemäße Hautreinigunsgsmittel auf der Haut belassen wird, hängt von dessen Verwendungszweck ab. Bei einer Handwaschpaste beträgt der Zeitraum 1 bis 2 Minuten, bei einer Gesichtsmaske 10 bis 30 Minuten und bei einem Pflaster mehrere Tage. Der Fachmann erkennt, daß bei einem Pflaster, insbesondere bei einem Sprühpflaster, die Entfernung des erfindungsgemäßen Hautreinigungsmittel auch gemeinsam mit dem Schorf erfolgen kann.

Das erfindungsgemäße Verfahren hat den Vorteil, daß es toxikologisch unbedenklich ist und die Umwelt weniger belastet.

Im folgenden wird die Erfindung anhand von Beispielen erläutert. Diese Erläuterungen sind lediglich beispielhaft und schränken den allgemeinen Erfindungsgedanken nicht ein.

### A. Handwaschpasten

### Prüfmethoden:

### 1. Hautverträglichkeit mit Hilfe des Duhring-Kammer-Testes:

Bei dieser Methode handelt es sich um ein in vivo-Testmodell zur Überprüfung der Hautverträglichkeit verschiedener Testprodukte in direktem Vergleich. Den 20 Probanden werden die zu testenden Produkte in luftdurchlässigen Aluminiumkammern (Finn-Chambers^{®} 18 mm Durchmesser) auf die volare Seite der Unterarme an vier Tagen hintereinander auf jeweils dasselbe Testareal appliziert. Die Applikationszeiten betragen zwei Stunden am ersten, vier Stunden am zweiten und jeweils sechs Stunden am dritten und vierten Tag. Die Befestigung der Finn-Chamber^{®} erfolgt mit Pflasterstreifen. Bei starken dermalen Effekten wird der Test für das jeweilige Testfeld auch vor erreichen der Gesamtapplikationszeit beendet. Beurteilt werden die entstandenen Hautirritationen nach der unten angegebenen Skala bzw. den Applikationszeiten.
R= Röttung (Erythem): 0= kein Erythem 4= starkes Erythem
S= Schuppung: 0= keine Schuppung 4= starke Schuppung
F=Fissuren: 0= keine Fissuren 4= starke Fissuren

Als Beurteilungskriterien ergeben sich die:
a. Irritaton als Mittelwert der Summe der Irritationswerte von R, S und F von n Probanden
b. Applikationszeit als Mittelwert der tolerierten Applikationszeiten in Stunden von n Probanden.

### 2. Prüfung der Reinigungsleistung

Mit mindestens acht Probanden werden zwei Produkte vergleichend geprüft. Voraussetzung ist, daß alle Probanden eine charakteristische, durch manuelle Arbeit bedingte Hautstruktur der Handinnenflächen besitzen. Morgens und nachmittags wird mit je einem Produkt folgender Test durchgeführt:
- eine definierte Menge Modellschmutz (0,2 bis 0,5 g) wird auf die Handinnenfläche und auf den Handrücken verteilt und 45 s verrieben
- 1 ½ Min. trocknen lassen
- eine definierte Menge des Testproduktes (0,3 bis 1,8 g) wird aufgetragen und eingerieben
- nach Antrocknen des Produktes wird das Produkt mit dem gebundenen Schmutz unter Reiben entfernt
- visuelle Beurteilung der Restverschmutzung (RV) auf dem Handrücken und der Handinnenfläche gemäß Skala s.u.
0 = sauber 5 = kein Reinigungseffekt (Abstufung in 0,5er Schritten möglich)
RVᵢₙₙₑₙ = Mittelwert der Restverschmutzung Handinnenflächen von n Meßreihen (Probanden)
RV_{außen} = Mittelwert der Restverschmutzung Handaußenflächen von n Meßreihen (Probanden)

Zusammenfassung eines für die Prüfung geeigneten Modellschmutzes:
Motoröl 54,15 %
Vaseline 18,05 %
Adeps lanae 18,05 %
Graphit 3,61 %
Flammruß 5,42 %
Eisenoxid (Fe₂O₃) 0,72 %

### 3. Toxikologische Prüfung

Die toxikologische Wertung der natürlichen und/oder synthetischen wasserbasierenden Latexemulsion kann durch die Bestimmung der Zytotoxizität und den Neutralrot-Test festgestellt werden.

Die Durchführung des Tests erfolgt nach der EURO-Norm EN 30 993-5, wobei unter Auslassung der 72h-Inkubationszeit die Prüfsubstanz in Zellkulturmedium verdünnt auf die Zellen appliziert werden.

Als Latexemulsion wurden ein Polyisopren-Latex mit der Bezeichnung KRATON^{®} IR-RP 401 der Shell AG eingesetzt.

| | | | |
|---|---|---|---|
| Zellen : | Balb/c 3T3 | Vehikel : | DMEM |
| Verdünnung : | 10% (w/v) | pH-Wert : | 8,22 |

### Neutralrot-Aufnahme:

| Konzentration (mg/ml) der Prüfsubstanz | 0 | 1 | 2,5 | 5 | 7,5 | 10 | 25 | 50 | 100 |
|---|---|---|---|---|---|---|---|---|---|
| Hemmung (%) der Neutralrot-Aufnahme | | -5,89 | -8,70 | 15,07 | 38,44 | 57,74 | 72,22 | 80,31 | 82,42 |

| | | |
|---|---|---|
| NR₅₀-Wert: | | W: 7,0 mg/ml (Vorversuch) |
| | 2. | V: 9,0 mg/ml (Versuch) |

### Bewertung:

Aus diesen Untersuchungen ergibt sich ein NR₅₀-Wert von 7,0 mg/ml bzw. 9,0 mg/ml. Das Produkt ist daher als schwach zytotoxisch einzustufen.

### Beispiele

Zur Prüfung der Reinigungsleistung und der Hautverträglichkeit wurden 9 Handwaschpasten hergestellt, deren Zusammensetzung der folgenden Tabelle 1 entnommen werden kann, wobei die Angaben in Gew.-% gemacht sind.

**Tabelle 1**

| Beispiel | 1 | 2 | 3 | 5 | 6 | 7 | 8 | 9 |
|---|---|---|---|---|---|---|---|---|
| Kraton IR 401 Latex | 17 | 30 | 50 | 80 | 80 | 30 | 30 | 30 |
| PPG-2-Methylether | 15 | 10 | 5 | 10 | 5 | | | |
| Di-n-butyladipat | | | | | | 10 | | |
| PEG 4 | | | | | | | 10 | |
| Hexylenglycol | | | | | | | | 10 |
| Isohexadecan | | | | | | | | |
| Pareth-5 | | 3 | 3 | 3 | | 3 | 3 | 3 |
| Laureth-6 | 1 | | | | 3 | | | |
| Wasser | 60 | 50 | 35 | 0 | 5 | 50 | 50 | 50 |
| Walnußschalenmehl | 6 | 6 | 6 | 6 | 6 | 6 | 6 | 6 |
| Verdicker | 1 | 1 | 1 | 1 | 1 | 1 | 1 | 1 |
| Gesamt | 100 | 100 | 100 | 100 | 100 | 100 | 100 | 100 |

| | | | | | | | | |
|---|---|---|---|---|---|---|---|---|
| Kraton^{™} IR 401 Latex = Isopren-Latex der Firma Shell Chemicals UT Ltd. PPG-2-Methylether = Dipropylenglykolmonomethylether PEG 4= Polyethylenglykol 400 P Pareth-5 = Polyethylenglykolether eines Gemisches aus synthetischem Cₐ-C₁₁-Fettalkohol (n = 5) Laureth-6 = Laurylalkoholpolyethylenglykolether (n = 6) Verdicker = Carbopol ETD 2020: Acrylsäure-Copolymer (98 - 100%) | | | | | | | | |

**Tabelle 2**

| Hautverträglichkeit | 0 | 0 | + | 0 | + | + | + | + |
|---|---|---|---|---|---|---|---|---|
| Hautreinigung im Vergleich zu Castrol Super Clean | + | + | 0 | + | 0 | + | 0 | 0 |

Alle in der Tabelle 1 angegebenen Substanzen wurden toxikologisch bewertet und als unproblematisch eingestuft.

Die Handwaschpasten gemäß den Beispielen 1-9 wurden mit Castrol Super Clean, einem Produkt der Castrol Ltd. verglichen. Die Ergebnisse des Vergleiches sind in Tabelle 2 zusammengefaßt, wobei 0 vergleichbar und + besser bedeutet.

## Patentansprüche

1. Hautreinigungsmittel enthaltend
- ein Polyisoprenlatex und
- ein Abrasivum.

2. Hautreinigungsmittel nach Anspruch 1, **dadurch gekennzeichnet, dass** der Gehalt an cis 1,4-Isopren im Polyisoprenlatex mindestens 90 Gew.-% beträgt.

3. Hautreinigungsmittel nach Anspruch 1 oder 2, **dadurch gekennzeichnet, dass** die Partikelgröße im Latex maximal 1,8 µm beträgt.

4. Hautreinigungsmittel nach Anspruch 1 oder 2, **dadurch gekennzeichnet, dass** der Polyisoprenlatex wasserbasierend ist.

5. Hautreinigungsmittel nach einem der Ansprüche 1 bis 4, **dadurch gekennzeichnet, dass** das Mittel eine Waschpaste und insbesondere eine Handwaschpaste ist.

6. Hautreinigungsmittel nach einem der Ansprüche 1 bis 5, **dadurch gekennzeichnet, dass** das Abrasivum gebleichtes Wallnussschalenmehl oder nichtquellbare Stärke ist.

7. Hautreinigungsmittel nach einem der Ansprüche 1 bis 6, **dadurch gekennzeichnet, dass** es ein organisches Lösemittel enthält ausgewählt aus der Gruppe bestehend aus
- Polyalkylenglykolen, Polyalkylenglykolethern und/oder Polyalkylenglykolestem;
- Mono- oder Polyestern einer gesättigten oder ungesättigten ein- oder mehrwertigen Carbonsäure mit 2 bis 30 Kohlenstoffatomen mit n- und iso-Alkanolen mit 2 bis 10 Kohlenstoffatomen; und
- aliphatischen Kohlenwasserstoffen mit C₁₆- bis C₂₀-Kohlenstoffatomen.

## Claims

1. A skin cleanser containing
- a polyisoprene latex and
- an abrasive.

2. A skin cleanser according to claim 1, **characterised in that** the content of cis-1,4-isoprene in the polyisoprene latex is at least 90 wt.%.

3. A skin cleanser according to claim 1 or claim 2, **characterised in that** the particle size in the latex is at most 1.8 µm.

4. A skin cleanser according to claim 1 or claim 2, **characterised in that** the polyisoprene latex is water-based.

5. A skin cleanser according to any one of claims 1 to 4, **characterised in that** the cleanser is a washing paste and in particular a hand washing paste.

6. A skin cleanser according to any one of claims 1 to 5, **characterised in that** the abrasive is bleached walnut shell flour or non-swellable starch.

7. A skin cleanser according to any one of claims 1 to 6, **characterised in that** it contains an organic solvent selected from the group consisting of
- polyalkylene glycols, polyalkylene glycol ethers and/or polyalkylene glycol esters;
- mono- or polyesters of a saturated or unsaturated mono- or polybasic carboxylic acid having 2 to 30 carbon atoms with n- and iso-alkanols having 2 to 10 carbon atoms; and
- aliphatic hydrocarbons having C₁₆ to C₂₀ carbon atoms.

## Revendications

1. Composition de nettoyage pour la peau comprenant
- un latex de polyisoprène et
- un abrasif.

2. Composition de nettoyage pour la peau selon la revendication 1, **caractérisée en ce que** la teneur en cis-1,4-isoprène dans le latex de polyisoprène est d'au moins 90 % en poids.

3. Composition de nettoyage pour la peau selon la revendication 1 ou 2, **caractérisée en ce que** la taille de particules dans le latex est d'au plus 1,8 µm.

4. Composition de nettoyage pour la peau selon la revendication 1 ou 2, **caractérisée en ce que** le latex de polyisoprène est à base d'eau.

5. Composition de nettoyage pour la peau selon l'une quelconque des revendications 1 à 4, **caractérisée en ce que** la composition est une pâte lavante et en particulier une pâte lavante pour les mains.

6. Composition de nettoyage pour la peau selon l'une quelconque des revendications 1 à 5, **caractérisée en ce que** l'abrasif est de la farine de coquilles de noix blanchie ou de l'amidon non gonflable.

7. Composition de nettoyage pour la peau selon l'une quelconque des revendications 1 à 6, **caractérisée en ce qu'**elle comprend un solvant organique choisi parmi le groupe constitué
- de polyalkylèneglycols, d'éthers de polyalkylèneglycols et/ou d'esters de polyalkylèneglycols ;
- de monoesters ou de polyesters d'un acide carboxylique monofonctionnel ou polyfonctionnel, saturé ou insaturé, renfermant de 2 à 30 atomes de carbone, avec des n-alcanols et des isoalcanols renfermant de 2 à 10 atomes de carbone; et
- d'hydrocarbures aliphatiques renfermant de 16 à 20 atomes de carbone.
